# EUROPÄISCHE PATENTSCHRIFT

(11) **EP 1 483 238 B1**
(45) Veröffentlichungstag und Bekanntmachung des Hinweises auf die Patenterteilung: **27.06.2007**
(21) Anmeldenummer: 03743348.9
(22) Anmeldetag: 28.02.2003
(51) Int. Cl.: C07D 207/26, C07D 307/08

(54) **VERFAHREN ZUR GLEICHZEITIGEN HERSTELLUNG VON TETRAHYDROFURAN UND PYRROLIDONEN**
METHOD FOR THE SIMULTANEOUS PRODUCTION OF TETRAHYDROFURANS AND PYRROLIDONES
PROCEDE DE PRODUCTION SIMULTANEE DE TETRAHYDROFURANE ET DE PYRROLIDONES

(30) Priorität: 02.03.2002 DE 10209633
(43) Veröffentlichungstag der Anmeldung: 08.12.2004
(73) Patentinhaber: BASF Aktiengesellschaft, 67056 Ludwigshafen (DE)
(72) Erfinder: FISCHER, Rolf-Hartmuth, 69121 Heidelberg (DE); RÖSCH, Markus, 55276 Dienheim (DE); BOTTKE, Nils, 68165 Mannheim (DE); WECK, Alexander, 67251 Freinsheim (DE); WINDECKER, Gunther, 67067 Ludwigshafen (DE); HESSE, Michael, 67549 Worms (DE); BORCHERT, Holger, 67591 Offstein (DE); SCHLITTER, Stephan, 67117 Limburgerhof (DE)
(86) Internationale Anmeldenummer: PCT/EP2003/002048
(87) Internationale Veröffentlichungsnummer: WO 2003/074482

(56) Entgegenhaltungen:
- EP-A- 0 443 392
- WO-A-01/85708
- WO-A-93/02068
- WO-A-97/24346

## Beschreibung

Die vorliegende Erfindung betrifft ein Verfahren zur gleichzeitigen Herstellung von reinem Tetrahydrofuran (THF) und Pyrrolidonen durch Hydrierung von C₄-Dicarbonsäuren und/oder deren Derivaten; bevorzugt Maleinsäure und ihren Derivaten, in Gegenwart von Kupfer-enthaltenden Katalysatoren zu Gemischen aus THF und gamma-Butyrolacton (GBL), destillative Auftrennung derselben in THF/Wassergemische und GBL enthaltende Gemische, Gewinnung von reinem THF und GBL durch weitere Destillation und schließlich Umsetzung von GBL mit Ammoniak oder primären Aminen zu Pyrrolidon(en).

Es ist aus JP-B 2.639.464 bekannt, Maleinsäureanhydrid (MSA) in der Gasphase in Gegenwart von Kupfer und Aluminiumoxid enthaltenden Katalysatoren zu Gemischen aus Butandiol und Tetrahydrofuran zu hydrieren. Als Einsatzstoff für die Hydrierung werden dabei in den Beispielen 1 - 4 und 7 Gemische aus MSA und GBL verwendet. Beispiel 5 dagegen beschreibt die Hydrierung von MSA zusammen mit Dioxan bei 210°C/15 bar in Gegenwart von Katalysatoren mit einem Kupfer- und Aluminiumgehalt von 36,7 % bzw. 17,7 % zu Gemischen aus 3,8 % Butandiol, 81,6 % THF und 14,1 % GBL. Beispiel 6 demonstriert die Hydrierung von MSA bei 220°C/60 bar in Gegenwart von Katalysatoren mit einem Kupfer- und Aluminiumgehalt von 28,5 % bzw. 24,5 %. Nachteilig an dem Ergebnis von Beispiel 5 ist, dass neben THF und GBL auch kleine, aber nicht vernachlässigbare Mengen an Butandiol entstehen, die von den Wertprodukten THF und GBL zusätzlich abgetrennt und aufgearbeitet werden müssen.

JP-B 2.639.463 zeigt, dass bei der Gasphasenhydrierung von MSA in Gegenwart von Kupfer, Zinkoxid und Aluminiumoxid enthaltenden Katalysatoren ebenfalls Gemische aus Butandiol und THF erhalten werden. Auch hier werden dabei in den Beispielen 1 - 6 und 10 Gemische aus MSA und GBL als Einsatzstoff verwendet. Beispiel 8 beschreibt die Hydrierung von MSA zusammen mit Dioxan bei 210°C/15 bar in Gegenwart von 16 % Kupfer, 35 % Zink und 9,6 % Aluminium enthaltenden Katalysatoren zu Tetrahydrofuran mit einer Ausbeute von 94,1 %. Butandiol und GBL wurden nicht gefunden. Dagegen wurden nach Beispiel 9 ausgehend von MSA bei 220°C/60 bar in Gegenwart von 18,3 % Kupfer, 36 % Zink und 8,6 % Aluminium enthaltenden Katalysatoren Hydrierausbeuten von 15,3 % Butandiol und 83,4 % THF ermittelt. Diese Angaben zeigen, dass das Verfahren nach JP 2.639.463 für die Herstellung von GBL und THF nicht geeignet ist.

WO 97/24.346 beschreibt die Gasphasen-Hydrierung von MSA in Gegenwart von Katalysatoren, die Kupfer, Aluminiumoxid und Binder enthalten, zu GBL. THF wird nicht beobachtet. So werden in Gegenwart eines Katalysators, der 84,6 % Kupferoxid, 9,9 % Aluminiumoxid und 5,5 % Graphit als Binder enthält, bei 275°C und Normaldruck, GBL-Ausbeuten von 98,2 % erzielt. Über einen Versuchszeitraum von 1600 Stunden betrug die GBL-Ausbeute 92 - 93 %. Die Anmeldung zeigt weiterhin, dass Chromoxid enthaltende Kupfer-Katalysatoren niedrige GBL-Ausbeuten bzw. schlechte KatalysatorStandzeiten liefern. Es entstehen außerdem beträchtliche Mengen an Bernsteinsäureanhydrid, das technische Probleme verursacht.

Der nach WO 97/24.346, Beispiel 2, erhaltene Hydrieraustrag wird nach Beispiel 5 ohne Aufarbeitung mit Methylamin zu N-Methylpyrrolidon umgesetzt. Hierzu wird der Wasser enthaltende Hydrieraustrag bei 290°C mit Methylamin erhitzt. Die gaschromatographisch ermittelte N-Methylpyrrolidon-Ausbeute beträgt 99,1 %. Aus dem erhaltenen Reaktionsaustrag muss NMP destillativ von Wasser, n-Butanol und gegebenenfalls anderen Nebenprodukten abgetrennt werden.

Die direkte Umsetzung des Hydrieraustrags mit Methylamin ist für ein Verfahren, das GBL und THF erzeugt, nachteilig. Sie führt dazu, dass das gebildete THF durch stickstoffhaltige Nebenprodukte verunreinigt werden kann. An die Reinheit von THF mit Faserqualität werden hohe Reinheitsanforderungen gestellt, da ein Großteil des THF zu Polytetrahydrofuran (PolyTHF), dem Vorprodukt der Spandex-Fasern, weiterverarbeitet wird. Die Umsetzung von GBL mit Ammoniak und Aminen zu Pyrrolidonen ist an sich bekannt und beispielsweise in Ullmann's Encyclopedia of Industrial Chemistry, 5. überarbeitete Ausgabe, Band A 22, Seiten 457 - 459 beschrieben.

Die Aufgabe der vorliegenden Erfindung bestand nun darin, ein Verfahren bereitzustellen, mit dem reines THF und Pyrrolidone gleichzeitig wirtschaftlich hergestellt werden können. Dazu ist es erforderlich, dass zunächst Gemische aus THF und GBL gewünschter Produktverhältnisse und hoher Gesamtausbeute an THF und GBL durch Hydrierung von C₄-Dicarbonsäuren und/oder deren Derivaten in Gegenwart geeigneter Katalysatoren hergestellt werden können. Diese Gemische aus THF und GBL sollten 5 - 95 Mol-% GBL und 95 - 5 Mol-% THF, bevorzugt 40 - 90 Mol-% GBL und 60 - 10 Mol-% THF, besonders bevorzugt 65 - 90 Mol.-% GBL und 35 - 10 Mol.-% THF enthalten. Weiterhin sollte der Katalysator mit MSA betrieben werden können, das nicht aufwendig vorgereinigt wurde, und trotzdem eine hohe Standfestigkeit aufweisen, also kein häufiges Regenerieren erfordern. Die erhaltenen Gemische aus THF und GBL sollten durch das erfindungsgemäße Verfahren effektiv und wirtschaftlich aufgetrennt werden können, um die Herstellung von reinem THF und Pyrrolidonen zu ermöglichen.

Diese Aufgabe wird gelöst in einem Verfahren zur gleichzeitigen Herstellung von gegebenenfalls alkylsubstutierte THF und Pyrrolidonen durch katalytische Hydrierung von C₄-Dicarbonsäuren und/oder deren Derivaten in der Gasphase in Gegenwart von Kupfer-enthaltenden Katalysatoren und Umsetzung von GBL mit Ammoniak oder primären Aminen zu Pyrrolidonen, dadurch gekennzeichnet, dass man
a) C₄-Dicarbonsäuren und/oder deren Derivate in der Gasphase bei 200 bis 300°C, 0,1 bis 100 bar, Katalysator-Belastungen von 0,01 bis 1 kg Edukt/l Katalysator * Stunde und Edukt/Wasserstoff-Molverhältnissen von 20 bis 800 in Gegenwart von Kupfer, Aluminium und/oder Zink enthaltenden Katalysatoren zu Gemischen aus THF und GBL hydriert,
b) den erhaltenen Hydrierautrag durch Destillation in ein THF/ Wasser-Gemisch als Kopfprodukt und ein GBL enthaltendes Sumpfprodukt auftrennt,
c) das THF/Wasser-Gemisch in einer aus drei Kolonnen bestehenden Destillationsanordnung trennt, in dem man Wasser aus dem Sumpf der ersten Kolonne abzieht, wasserhaltiges THF von der zweiten in die erste Kolonne zurückführt, einen Seitenstrom der ersten in die zweite Kolonne leitet, das Sumpfprodukt der dritten Kolonne in die erste Kolonne zurückführt, am Kopf der ersten Kolonne ein Destillat entnimmt, wobei ein Seitenabzug der zweiten Kolonne in die dritte Kolonne geleitet wird und das reine THF als Kopfprodukt der dritten Kolonne erhält,
d) aus dem GBL enthaltenden Sumpfprodukt aus Stufe b) durch Destillation GBL gewinnt und
e) das so erhaltene GBL mit Ammoniak oder Aminen zu entsprechenden Pyrrolidonen umsetzt.

Unter dem Begriff C₄-Dicarbonsäuren und deren Derivate werden im Bezug auf die vorliegende Anmeldung Maleinsäure und Bernsteinsäure, die gegebenenfalls einen oder mehrere C₁-C₆-Alkylsubstituenten aufweisen sowie die Ester und Anhydride dieser gegebenenfalls alkylsubstituierten Säuren verstanden. Ein Beispiel einer solchen Säure ist Citraconsäure. Vorzugsweise werden die jeweiligen Anhydride einer gegebenen Säure eingesetzt. Bevorzugt sind Maleinsäure und/oder ihre Derivate. Insbesondere ist das verwendete Edukt Maleinsäureanhydrid (MSA). In dem erfindungsgemäßen Verfahren wird bevorzugt MSA, das durch Oxidation von Benzol, C₄-Olefinen oder n-Butan hergestellt wurde, wobei das durch Oxidation erhaltene Roh-MSA mit einem Lösungsmittel aus dem Rohproduktgemisch extrahiert und anschließend aus diesem Lösungsmittel mit Wasserstoff ausgetrieben wurde, eingesetzt.

Das erfindungsgemäße Verfahren zeichnet sich dadurch aus, dass es kostengünstig betrieben werden kann, hohe THF + GBL-Gesamtausbeuten von 95 % und mehr erreicht, und durch eine einfache Aufarbeitung spezifikationsgerechtes THF und nach Umsetzung des GBL mit Ammoniak oder Aminen spezifikationsgerechte Pyrrolidone erhalten werden. Durch die gleichzeitige Herstellung von THF und Pyrrolidonen ist der Bau großer und damit besonders wirtschaftlicher Produktionsanlagen ("economy of scale") möglich.

Das Verfahren kann diskontinuierlich oder kontinuierlich, bevorzugt kontinuierlich durchgeführt werden.

Die Hydrierung der C₄-Dicarbonsäuren und ihrer Derivate wird erfindungsgemäß so durchgeführt, dass Gemische aus THF und GBL mit einem GBL-Gehalt von 5 bis 95 Mol-%, bezogen auf C₄-Dicarbonsäuren und/oder ihrer Derivate, bevorzugt 40 - 90 Mol-%, besonders bevorzugt 65 bis 90 Mol-% erhalten werden. Die zu 100 % fehlenden Werte stellen die jeweiligen THF-Gehalte dar.

Ein wichtiger Aspekt der Stufe a) des erfindungsgemäßen Verfahrens ist die Wahl des Katalysators, der als katalytisch aktiven Hauptbestandteil Kupferoxid aufweist. Dieses ist auf einem oxidischen Träger, der eine geeignete Anzahl an sauren Zentren besitzen muss, aufgebracht. Die erforderliche Menge an oxidischem Träger hängt von der darin enthaltenen Menge an sauren Zentren ab. Ein geeignetes Trägermaterial mit einer ausreichenden Zahl an sauren Zentren ist Aluminiumoxid, dessen Verwendung gemäß einer Ausführungsform der vorliegenden Erfindung bevorzugt ist. Gemäß einer anderen Ausführungsform der vorliegenden Erfindung ist es bevorzugt, als saures Trägermaterial eine Kombination von Aluminiumoxid mit Zinkoxid im Gew.-Verhältnis 20:1 bis 1:20, vorzugsweise 5:1 bis 1:5 zu verwenden. Für Materialien, die eine große Menge solcher sauren Zentren aufweisen, liegt die untere Grenze der Menge an aus einem solchen Material bestehendem Träger bei 20 Gew.-%. Die Menge an Kupferoxid liegt bei Werten von < 80 Gew.-%. Bevorzugte Katalysatorzusammensetzungen weisen < 70 Gew.-% Kupferoxid und > 30 Gew.-% Träger, besonders bevorzugte Katalysatoren 10 bis 65 Gew.-% Kupferoxid und 35 bis 90 Gew.-% Träger auf.

Niedrige Kupferoxid-Gehalte sind auch aufgrund des damit erzielten Kostenvorteils bevorzugt. Durch die sauren Trägermaterialien lassen sich hohe Ausbeuten erzielen. Die erfindungsgemäß verwendeten Katalysatoren können Chrom enthalten, sind jedoch bevorzugt chromfrei.

Die eingesetzten Katalysatoren können zudem ein Hilfsmittel in einer Menge von 0 bis 10 Gew.-% enthalten. Unter Hilfsmittel versteht man organische und anorganische Stoffe, die zu einer verbesserten Verarbeitung während der Katalysatorherstellung und/ oder zu einer Erhöhung der mechanischen Festigkeit der Katalysatorformkörper beitragen. Derartige Hilfsmittel sind dem Fachmann bekannt; Beispiele umfassen Graphit, Stearinsäure, Kieselgel und Kupferpulver.

Die Katalysatoren lassen sich nach dem Fachmann bekannten Methoden herstellen. Bevorzugt sind Verfahren, bei denen das Kupferoxid fein verteilt und innig vermischt mit den anderen Bestandteilen anfällt, besonders bevorzugt sind Fällungsreaktionen. Dabei werden in einem Lösungsmittel gelöste Vorläuferverbindungen in Gegenwart weiterer löslicher oder im Lösungsmittel suspendierter Metallverbindungen mit einem Fällungsmittel ausgefällt, abfiltriert, gewaschen, getrocknet und gegebenenfalls calciniert.

Diese Ausgangsmaterialien können nach bekannten Methoden zu den Formkörpern verarbeitet werden, beispielsweise Extrudieren, Tablettieren oder durch Agglomerationsverfahren, gegebenenfalls unter Zusatz von Hilfsmitteln.

Alternativ können erfindungsgemäße Katalysatoren beispielsweise auch durch Aufbringen der Aktivkomponente auf einen Träger hergestellt werden, beispielsweise durch Tränken oder Aufdampfen. Weiterhin können erfindungsgemäße Katalysatoren durch Verformen einer heterogenen Mischung aus Aktivkomponente oder Vorläuferverbindung hiervon mit einer Trägerkomponente oder Vorläuferverbindung hiervon erhalten werden.

Bei der erfindungsgemäßen Hydrierung, bei der neben MSA andere, vorstehend definierte C₄-Dicarbonsäuren oder deren Derivate als Edukt eingesetzt werden können, wird der Katalysator in reduzierter, aktivierter Form verwendet. Die Aktivierung erfolgt mit reduzierenden Gasen, vorzugsweise Wasserstoff oder Wasserstoff/Inertgas-Gemischen, entweder vor oder nach dem Einbau in den Reaktor, in dem das erfindungsgemäße Verfahren durchgeführt wird.

Wurde der Katalysator in oxidischer Form in den Reaktor eingebaut, so kann die Aktivierung sowohl vor dem Anfahren der Anlage mit der erfindungsgemäßen Hydrierung als auch während des Anfahrens, also in situ, durchgeführt werden. Die separate Aktivierung vor dem Anfahren der Anlage erfolgt im allgemeinen mit reduzierenden Gasen, vorzugsweise Wasserstoff oder Wasserstoff/Inertgas-Gemischen bei erhöhten Temperaturen, vorzugsweise zwischen 100 und 300°C. Bei der sogenannten in-situ-Aktivierung erfolgt die Aktivierung beim Hochfahren der Anlage durch Kontakt mit Wasserstoff bei erhöhter Temperatur.

Die Katalysatoren werden als Formkörper verwendet. Beispiele umfassen Stränge, Rippstränge, andere Extrudatformen, Tabletten, Ringe, Kugeln und Splitt.

Die BET-Oberfläche der Kupferkatalysatoren beträgt im oxidischen Zustand 10 bis 400 m²/g, vorzugsweise 15 bis 200 m²/g, insbesondere 20 bis 150 m²/g. Die Kupferoberfläche (N₂O-Zersetzung) des reduzierten Katalysators beträgt im Einbauzustand > 0,2 m²/g, vorzugsweise > 1 m²/g, insbesondere > 2 m²/g.

Gemäß einer Variante der Erfindung werden Katalysatoren verwendet, die eine definierte Porosität aufweisen. Diese Katalysatoren zeigen als Formkörper ein Porenvolumen von ≥ 0,01 ml/g für Porendurchmesser > 50 nm, vorzugsweise ≥ 0,025 ml/g für Porendurchmesser > 100 nm und insbesondere ≥ 0,05 ml/g für Porendurchmesser > 200 nm. Weiterhin liegt das Verhältnis von Makroporen mit einem Durchmesser > 50 nm zum Gesamtporenvolumen für Poren mit einem Durchmesser > 4 nm bei Werten > 10 %, bevorzugt > 20 %, insbesondere > 30 %. Oftmals lassen sich durch Verwendung dieser Katalysatoren hohe THF + GBL-Ausbeuten erreichen. Die erwähnten Porositäten wurden durch Quecksilber-Intrusion nach DIN 66133 bestimmt. Es wurden die Daten im Porendurchmesserbereich von 4 nm bis 300 µm ausgewertet.

Die erfindungsgemäß verwendeten Katalysatoren besitzen im allgemeinen eine ausreichende Standzeit. Für den Fall, dass die Aktivität und/oder Selektivität des Katalysators dennoch im Laufe ihrer Betriebszeit sinken sollte, kann dieser durch dem Fachmann bekannte Maßnahmen regeneriert werden. Hierzu zählt vorzugsweise eine reduktive Behandlung des Katalysators im Wasserstoffstrom bei erhöhter Temperatur. Gegebenenfalls kann der reduktiven Behandlung eine oxidative vorausgehen. Hierbei wird die Katalysatorschüttung mit einem molekularen Sauerstoff enthaltenden Gasgemisch, beispielsweise Luft, bei erhöhter Temperatur durchströmt. Weiterhin besteht die Möglichkeit, den Katalysator mit einem geeigneten Lösungsmittel, beispielsweise Ethanol, THF oder GBL, zu waschen und anschließend in einem Gasstrom zu trocknen.

Zum Erzielen der erfindungsgemäßen THF + GBL-Ausbeuten ist weiterhin das Einhalten gewisser Reaktionsparameter erforderlich.

Ein wichtiger Parameter ist das Einhalten einer geeigneten Reaktionstemperatur. Dies wird zum einen erreicht durch eine genügend hohe Eingangstemperatur der Edukte. Diese liegt bei Werten von > 200 bis 300°C, vorzugsweise 235 bis 280°C. Um eine akzeptable bzw. hohe THF + GBL-Selektivität und -Ausbeute zu erhalten, muss die Reaktion so durchgeführt werden, dass am Katalysatorbett, an dem die eigentliche Reaktion stattfindet, eine geeignet hohe Reaktionstemperatur herrscht.

Das Wasserstoff/Edukt-Molverhältnis ist ebenfalls ein Parameter, der einen wichtigen Einfluss auf die Produktverteilung und auch die Wirtschaftlichkeit des erfindungsgemäßen Verfahrens hat. Aus wirtschaftlicher Sicht ist ein niedriges vlasserstoff/Edukt-Verhältnis wünschenswert. Die Untergrenze liegt bei einem Wert von rund 5, wobei jedoch generell höhere Wasserstoff/Edukt-Molverhältnisse von 20 bis 800 angewendet werden.

Um die erfindungsgemäß verwendeten Wasserstoff/Edukt-Molverhältnisse einzustellen, wird ein Teil, vorteilhafterweise die Hauptmenge, des Wasserstoffs im Kreis gefahren. Hierzu setzt man im allgemeinen die dem Fachmann bekannten Kreisgasverdichter ein. Die chemisch durch die Hydrierung verbrauchte Wasserstoffmenge wird ergänzt. In einer bevorzugten Ausführungsform wird ein Teil des Kreisgases ausgeschleust, um Inertverbindungen, beispielsweise n-Butan, zu entfernen. Der im Kreis geführte Wasserstoff kann auch, gegebenenfalls nach Vorheizen, zum Verdampfen des Eduktstroms benutzt werden.

Auch der Volumenstrom der Reaktionsgase, generell ausgedrückt als GHSV (Gas Hourly Space Velocity) ist eine wichtige Größe des erfindungsgemäßen Verfahrens. Die GHSV-Werte des erfindungsgemäßen Verfahrens liegen bei Werten von 100 bis 10.000 Nm³/m³h, vorzugsweise 1000 bis 3000 Nm³/m³h, insbesondere 1100 bis 2500 Nm³/m³h.

Der Druck, bei dem die erfindungsgemäße Hydrierung durchgeführt wird, liegt bei Werten von 0,1 bis 100 bar, vorzugsweise 2 bis 30 bar, insbesondere 3 bis 20 bar.

Gemeinsam mit dem Wasserstoff-Kreisgas werden alle Produkte im Kreis geführt, die beim Kühlen des aus dem Hydrierreaktor austretenden Gasstroms nicht oder nicht vollständig auskondensieren. Dies sind vor allem THF, Wasser und Nebenprodukte wie Methan und Butan. Die Kühltemperatur beträgt 0 bis 60°C, vorzugsweise 20 bis 45°C. Der THF-Gehalt des Kreisgases beträgt 0,1 bis 5 Vol.-%, insbesondere 1 bis 3 Vol.-%.

Bei der Hydrierung von MSA zu THF werden der Reihe nach die Zwischenprodukte Bernsteinsäureanhydrid (BSA) und GBL durchlaufen. Das erfindungsgemäße Verfahren macht es möglich, MSA und Derivate zu Reaktionsgemischen zu hydrieren, die 5 - 95 Mol-% THF und entsprechend 95 - 5 Mol-% GBL (bezogen auf MSA) enthalten. Das GBL/ THF-Verhältnis lässt sich vor allem durch die Wahl des Hydrierkatalysators, die Katalysator-Belastung, die Hydriertemperatur, den Reaktionsdruck und das Ausgangsprodukt/Wasserstoff-Molverhältnis beeinflussen. Der THF-Gehalt der GBL/THF-Gemische steigt mit zunehmender Anzahl an sauren Katalysator-Zentren, mit abnehmender Katalysator-Belastung, steigender Temperatur und steigendem Reaktionsdruck. Durch eine geringe Zahl von Vorversuchen können die Reaktionsbedingungen für das jeweils gewünschte GBL/THF-Molverhältnis ermittelt werden.

Aus der Literatur ist bekannt, dass THF und GBL mit Wasserstoff in Gegenwart von Kupfer-Katalysatoren zu n-Butanol hydriert werden. Dabei zeichnet sich das erfindungsgemäße Verfahren dadurch aus, dass trotz der hohen THF-Anteile im Kreisgas, die generell leicht weiter zu n-Butanol hydriert werden, THF + GBL-Ausbeuten von über 90 %, teilweise auch über 95 %, erzielt werden. Als Reaktortypen kommen alle, für heterogen katalysierte Reaktionen mit einem gasförmigen Edukt- und Produktstrom geeigneten Apparate in Betracht. Bevorzugt sind Rohrreaktoren, Schachtreaktoren oder Reaktoren mit innerer Wärmeabfuhr, beispielsweise Rohrbündelreaktoren, es ist auch der Einsatz eines Wirbelbetts möglich. Besonders bevorzugt eingesetzt werden Rohrbündelreaktoren. Es können mehrere Reaktoren parallel oder hintereinander geschaltet eingesetzt werden. Prinzipiell kann zwischen die Katalysatorbetten eine Zwischeneinspeisung erfolgen. Möglich ist auch eine Zwischenkühlung zwischen oder in den Katalysatorbetten. Bei Einsatz von Festbettreaktoren ist eine Verdünnung des Katalysators durch Inertmaterial möglich.

Der aus dem Reaktor austretende Gasstrom wird auf 10 bis 60°C gekühlt. Dabei werden die Reaktionsprodukte auskondensiert und in einen Abscheider geleitet. Der nicht-kondensierte Gasstrom wird vom Abscheider abgezogen und dem Kreisgasverdichter zugeführt. Eine kleine Kreisgasmenge wird ausgeschleust. Die auskondensierten Reaktionsprodukte werden dem System kontinuierlich entnommen und der Aufarbeitung zugeführt. Als Nebenprodukte findet man in der auskondensierten Flüssigkeitsphase hauptsächlich Wasser und n-Butanol neben geringen Mengen an Propanol, Methanol, Ethanol, n-Butyraldehyd, Butylmethylether und weiteren Sauerstoff enthaltenden Verbindungen.

Das erfindungsgemäße Verfahren zeichnet sich dadurch aus, dass zu hydrierende Edukte unterschiedlicher Reinheit in die Hydrierreaktion eingesetzt werden können. Selbstverständlich kann ein Edukt hoher Reinheit, insbesondere MSA, in die Hydrierreaktion eingesetzt werden. Der erfindungsgemäß verwendete Katalysator sowie die sonstigen erfindungsgemäß gewählten Reaktionsbedingungen ermöglichen aber auch den Einsatz von Edukten, insbesondere MSA, das mit den üblichen, bei der Oxidation von Benzol, Butenen oder n-Butan anfallenden Verbindungen sowie eventuell weiteren Komponenten verunreinigt ist. Somit kann das erfindungsgemäße Hydrierverfahren in einer weiteren Ausführungsform eine vorgeschaltete Stufe umfassen, die das Herstellen des zu hydrierenden Edukts durch partielle Oxidation eines geeigneten Kohlenwasserstoffs sowie das Abtrennen des zu hydrierenden Edukts aus dem damit erhaltenen Produktstrom umfasst.

Insbesondere ist dieses zu hydrierende Edukt MSA. Dabei wird bevorzugt MSA eingesetzt, welches aus der Partialoxidation von Kohlenwasserstoffen stammt. Geeignete Kohlenwasserstoffströme sind Benzol, C₄-Olefine (z.B. n-Butene, C₄-Raffinatströme) oder n-Butan. Besonders bevorzugt eingesetzt wird n-Butan, da es einen preiswerten, wirtschaftlichen Einsatzstoff darstellt. Verfahren zur Partialoxidation von n-Butan sind beispielsweise in Ullmann's Encyclopedia of Industrial Chemistry, 6^{th} Edition, Electronic Release, Maleic and Fumaric Acids - Maleic Anhydride beschrieben.

Der so erhaltene Reaktionsaustrag wird dann in einem geeigneten organischen Lösungsmittel oder -Gemisch aufgenommen, das bei Atmosphärendruck einen um mindestens 30°C höheren Siedepunkt als MSA hat.

Dieses Lösungsmittel (Absorptionsmittel) wird auf eine Temperatur im Bereich zwischen 20 und 160°C, bevorzugt zwischen 30 und 80°C, gebracht. Der Maleinsäureanhydrid enthaltende Gasstrom aus der Partialoxidation kann in vielfältiger Weise mit dem Lösungsmittel in Kontakt gebracht werden: (i) Einleiten des Gasstroms in das Lösungsmittel (z.B. über Gaseinleitungsdüsen oder Begasungsringe), (ii) Einsprühen des Lösungsmittels in den Gasstrom und (iii) Gegenstromkontakt zwischen dem nach oben strömenden Gasstrom und dem nach unten strömenden Lösungsmittel in einer Boden- oder Packungskolonne. In allen drei Varianten können die dem Fachmann bekannten Apparate zur Gasabsorption eingesetzt werden. Bei der Wahl des einzusetzenden Lösungsmittels ist darauf zu achten, dass dies nicht mit dem Edukt, beispielsweise dem vorzugsweise eingesetzten MSA, reagiert. Geeignete Lösungsmittel sind: Trikresylphosphat, Dibutylmaleat, hochmolekulare Wachse, aromatische Kohlenwasserstoffe mit einem Molekulargewicht zwischen 150 und 400 und einem Siedepunkt oberhalb 140°C, wie beispielsweise Dibenzylbenzol; Dialkylphthalate mit C₁-C₈-Alkylgruppen, beispielsweise Dimethylphthalat, Diethylphthalat, Dibutylphthalat, Di-n-Propyl-und Di-iso-Propyl-phthalat; Di-C₁-C₄-Alkylester anderer aromatischer, aliphatischer und cycloaliphatischer Dicarbonsäuren, beispielsweise Dimethyl-2,3-Naphthalin-Dicarbonsäure, Dimethyl-1,4-Cyclohexan-Dicarbonsäure, Methylester langkettiger Fettsäuren mit beispielsweise 14 bis 30 Kohlenstoffatomen, hochsiedende Ether, beispielsweise Dimethylether von Polyethylenglykol, beispielsweise Tetraethylenglykoldimethylether.

Die nach der Behandlung mit dem Absorptionsmittel resultierende Lösung hat generell einen MSA-Gehalt von etwa 5 bis 400 Gramm pro Liter.

Der nach der Behandlung mit dem Absorptionsmittel verbleibende Abgasstrom enthält hauptsächlich die Nebenprodukte der vorangegangenen Partialoxidation, wie Wasser, Kohlenmonoxid, Kohlendioxid, nicht umgesetzte Butane, Essig- und Acrylsäure. Der Abgasstrom ist praktisch frei von MSA.

Anschließend wird das gelöste MSA aus dem Absorptionsmittel ausgetrieben. In der Strippkolonne wird ein Temperaturprofil beobachtet, das sich aus den Siedepunkten von MSA am Kopf und dem nahezu MSA-freien Absorptionsmittel am Sumpf der Kolonne bei dem jeweiligen Kolonnendruck und der eingestellten Verdünnung mit Trägergas ergibt.

Um Verluste an Lösungsmittel zu verhindern, können sich oberhalb der Zufuhr des Roh-MSA-Stromes Rektifiziereinbauten befinden. Das vom Sumpf abgezogene, nahezu MSA-freie Absorptionsmittel wird wieder der Absorptionszone zugeführt. Im Fall der Direktstrippung mit Wasserstoff wird vom Kopf der Kolonne ein nahezu gesättigter Gasstrom von MSA in Wasserstoff abgezogen. Im anderen Fall wird das kondensierte MSA in einen Verdampfer gepumpt und dort in den Kreisgasstrom verdampft.

Der MSA-Wasserstoff-Strom enthält noch Nebenprodukte, die bei der partiellen Oxidation von n-Butan, Butenen oder Benzol mit Sauerstoff enthaltenden Gasen entstehen, sowie nicht abgetrenntes Absorptionsmittel. Hierbei handelt es sich vor allem um Essigsäure und Acrylsäure als Nebenprodukte, Wasser, Maleinsäure sowie die als Absorptionsmittel verwendeten Verbindungen. Das MSA enthält Essigsäure in Mengen von 0,01 bis 1 Gew.-%, vorzugsweise 0, 1 bis 0,8 Gew.-% und Acrylsäure in Mengen von 0,01 bis 1 Gew.-%, vorzugsweise 0,1 bis 0,8 Gew.-%, bezogen auf MSA. In der Hydrierstufe werden Essigsäure und Acrylsäure ganz oder teilweise zu Ethanol bzw. Propanol hydriert. Der Maleinsäure-Gehalt beträgt 0,01 bis 1 Gew.-%, insbesondere 0,05 bis 0,3 Gew.-%, bezogen auf MSA.

Werden Dialkylphthalate als Absorptionsmittel eingesetzt, hängt deren Gehalt im MSA stark vom richtigen Betrieb der Strippkolonne, insbesondere vom Verstärkungsteil ab. Phthalatgehalte von bis 1,0 Gew.-%, insbesondere bis 0,5 Gew.-% sollten bei geeigneter Betriebsweise nicht überschritten werden, da sonst der Verbrauch an Absorptionsmittel zu hoch wird.

Der so erhaltene Wasserstoff/Maleinsäureanhydrid-Strom wird nun der Hydrierzone zugeführt und wie oben beschrieben hydriert. Die Katalysatoraktivität und -standzeit ist dabei verglichen mit dem Einsatz von stark, beispielsweise durch Destillation, vorgereinigtem MSA praktisch unverändert. Das erfindungsgemäße Verfahren erlaubt THF + GBL-Ausbeuten, die bei Werten von etwa 90 %, günstigenfalls etwa 95 %, liegen. Es wird dabei auch eine hohe Produktselektivität erzielt.

Der auskondensierte Hydrieraustrag wird in Stufe b) destillativ in ein THF/Wasser-Gemisch als Kopfprodukt und ein GBL enthaltendes Sumpfprodukt getrennt. Dabei werden in einer Destillationskolonne bei einem Druck von 1,3 absolut, Kopftemperaturen von 70 bis 80°C und Sumpftemperaturen von 200 bis 220°C eingestellt.

Reines THF durch eine Destillation in drei Kolonnen zu gewinnen, ist aus DE 3.726.805 bekannt. In den ersten beiden Kolonnen wird durch Zweidruckdestillation das THF/Wasser-Azeotrop gebrochen, in der dritten Kolonne erfolgt die THF-Reindestillation. Das Sumpfprodukt der ersten Kolonne enthält, neben weiteren gegenüber THF hochsiedenden Nebenprodukten das gesamte Rest-GBL. Die wasserhaltigen THF-Rohprodukte, insbesondere die THF-Wasser-Azeotrope, der Gasphasenhydrierung von MSA werden jedoch durch das aus DE 37 26 805 bekannte Verfahren nicht ausreichend aufgereinigt, um die insbesondere bei der Weiterbearbeitung des THF's, beispielsweise zu PTHF, an THF gestellten Reinheitsanforderungen zu erfüllen.

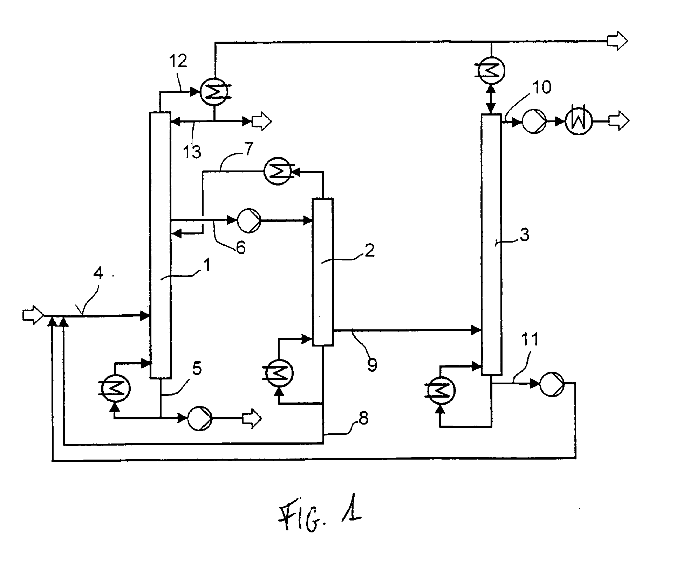

Nach dem neuen Verfahren, das Gegenstand der parallelen deutschen Anmeldung der Anmelderin mit dem Titel "Verfahren zur destillativen Reinigung von THF" ist, nimmt man die destillative Reinigung des rohen wasserhaltigen THF-Wasser-Azeotrops in Stufe c) des erfindungsgemäßen Verfahrens, in drei hintereinandergeschalteten Destillationskolonnen so vor, wie es aus der Figur ersichtlich ist. Dabei werden die Kolonnen auf an sich übliche Weise betrieben, und zwar die erste Kolonne (1) mit Druck von 1,3 bar mit mindestens 10, bevorzugt 30 bis 70, besonders bevorzugt 45 bis 55 theoretischen Trennstufen und einem Rückflussverhältnis, bezogen auf den Seitenabzug (6) von 0,5 bis 5, die zweite Kolonne (2) mit mindestens 10, bevorzugt 30 bis 70, besonders bevorzugt 55 bis 45, theoretischen Trennstufen, einem Druck von 5 bis 10 bar, bevorzugt zwischen 7 und 9 bar, besonders bevorzugt 8 bar, und die dritte Kolonne (3) mit mindestens 10 theoretischen Trennstufen, bevorzugt 30 bis 70 Trennstufen, besonders bevorzugt 45 bis 55 theoretischen Trennstufen, bei einem Druck von 0,9 bis 2 bar, bevorzugt 1 bis 1,5 bar und besonders bevorzugt 1,3 bar und einem Rückflussverhältnis von etwa 3,8.

Jede der drei Kolonnen (1), (2) und (3) weist mindestens eine theoretische Trennstufe auf, welche dadurch gekennzeichnet ist, das der entstandene Brüdenstrom und der flüssige Rücklauf der Stufe im Gegenstromprinzip aneinander vorbeigeführt werden. Die Einbauten der Kolonnen (1), (2) und (3) können aus Füllkörpern, Gewebe- oder Blechpackungen oder Trennböden wie Ventil-, Tunnel- oder Siebböden bestehen. Eine Definition für eine theoretische Trennstufe kann beispielsweise E.-U. Schlunder, F. Thurner, Destillation, Absorption, Extraktion, Thieme Verlag 1986, Seite 66 und Seiten 131 - 132 entnommen werden.

Das rohe THF/Wasser-Gemisch aus Stufe b), das nach a) durch Gasphasenhydrierung von MSA und anschließende Abtrennung des GBL's gemäß b) gewonnen wurde, besteht im allgemeinen aus wechselnden Mengen THF als Hauptprodukt, 10 bis 20 Gew.-% n-Butanol (n-BuOH), 0,1 bis 1 Gew.-% Methanol (MeOH), Ethanol (EtOH) und Propanol (ProOH), 100 bis 500 ppm Gamma-Butyrolacton (GBL), etwa 100 ppm Butyraldehyd (BA) und Butylmethylether (BME), weiteren Sauerstoff-funktionalisierten CH-Verbindungen in Konzentrationen < 200 ppm, sowie Wasser.

Es wird seitlich über die Zuleitung (4) in die erste Kolonne geleitet. Die Zuleitung (4) befindet sich zweckmäßigerweise in der unteren Hälfte oberhalb des Sumpfes der Kolonne. Erfindungsgemäß wurde erkannt, dass der Zulauf zwischen der 1. und 30. theoretischen Trennstufe, bevorzugt zwischen der 1. und 20. Trennstufe, besonders bevorzugt zwischen der 1. und 10. Trennstufe liegen sollte. Vom Sumpf der Kolonne werden Wasser und schwersiedende Komponenten, welche einen höheren Siedepunkt als THF aufweisen, wie GBL, Ethanol, Propanol, Butanol zusammen mit Wasser, ausgeschleust (5). Die Leichtsieder, welche einen höheren Siedepunkt als THF aufweisen, wie Methanol, werden über Leitung (12) mit THF über Kopf gezogen, teilweise über einen Wärmetauscher kondensiert und als Rücklauf (13) wieder in Kolonne (1) gefahren. Die Kolonne (1) weist einen oberhalb der Zuleitung (4) angebrachten Seitenabzug (6) auf, durch den man ein bevorzugt flüssiges THF/Wasser-Gemisch, das jedoch gasförmig oder als Flüssigkeits/Gasgemisch vorliegen kann, entnimmt und unter Druckerhöhung über eine Pumpe seitlich in die Mitteldruckkolonne (2) einleitet.

Der Seitenabzug (6) ist zwischen der 20. und 70. theoretischen Trennstufe der Kolonne (1), bevorzugt zwischen der 30 und 55 Trennstufe, besonders bevorzugt der 30. und 40. Trennstufe angeordnet.

In dem Seitenabzug (6) liegt THF zum Wasser in einem Gewichtsverhältnis von 13:1 bis 25:1, bevorzugt in einem Verhältnis von 15:1 bis 22:1 vor.

Der Zulauf des Seitenabzuggemisches (6) erfolgt im oberen Teil der Mitteldruckkolonne (2), zwischen der 30. und 70. theoretischen Trennstufe, bevorzugt zwischen der 40. und 60. Trennstufe, besonders bevorzugt zwischen der 50. und 60. Trennstufe. Unter Verschiebung des azeotropen Punktes des THF/Wassergemisch wird in der Mitteldruckkolonne (2) das Gemisch nochmals aufgetrennt. Vom Kopf dieser Mitteldruckkolonne wird wasserreiches THF über einen Wärmetauscher kondensiert und zwischen Seitenabzug (6) und dem Kolonnensumpf in die erste Kolonne zurückgeführt (7). Das Sumpfprodukt dieser Mitteldruckkolonne (2), das im wesentlichen wasserfrei ist, und aus THF und Schwersiedern wie beispielsweise Butyraldehyd, Butylmethylether und weiteren hochsiedende Sauerstoff-funktionalisierten CH-Verbindungen besteht, wird über Leitung (8) zurückgeführt und kann mit rohem wasserhaltigen THF vermischt, erneut Kolonne (1) über Leitung (4) als Feed zugeführt werden.

Im Abtriebsteil der Mitteldruckkolonne (2) reichert sich THF auf über 99 Gew.-% an. Um die wasserdampfflüchtigen Komponenten nicht in die Kolonne (3) zu schleppen, wird ein THF-reicher Strom in bevorzugt flüssiger Form, der jedoch auch gasförmig oder als Flüssigkeits/Gasgemisch vorliegen kann, zwischen der Dampfphase des Sumpfes und der Hälfte der theoretischen Kolonnenstufenzahl an einem flüssigen Seitenabzug (9) kurz über dem Sumpf der Mitteldruckkolonne (2) entnommen.

Der der Kolonne (2) entnommene Seitenabzug (9) enthält 50 bis 100 Gew.-% THF, bevorzugt 80 bis 100 Gew.-%, besonders bevorzugt 95 bis 100 Gew.-% THF.

Der Seitenabzug (9) wird oberhalb des Sumpfes in Kolonne (3) eingespeist. Die Zulaufstelle liegt zwischen der 1. und 30. theoretischen Trennstufe, bevorzugt zwischen der 1. und 15. Trennstufe, besonders bevorzugt zwischen 5. und 10. Trennstufe. Reines THF wird am Kopf der Kolonne (10) in flüssiger oder gasförmiger Form oder als Flüssigkeits/Gasgemisch, bevorzugt flüssig, abgezogen, während das flüssige Sumpfprodukt (11) über eine Pumpe in Kolonne (1) rückgeführt wird. Das erhaltene reine THF ist insbesondere für die Weiterverarbeitung zu PolyTHF und Spandex-Fasern geeignet.

Das als Sumpfprodukt in Stufe b) anfallende GBL-enthaltende Produkt fällt als Gemisch von Wasser, n-Butanol und GBL an und kann in an sich bekannter Weise in Anwesenheit oder Abwesenheit von Katalysatoren direkt, das heißt ohne weitere Aufarbeitung, mit Ammoniak oder Aminen zu Pyrrolidonen umgesetzt werden, die wiederum destillativ aufgereinigt werden können. Es ist jedoch bevorzugt, das GBL-enthaltende Sumpfprodukt zunächst destillativ aufzureinigen. Die Umsetzung von GBL mit Ammoniak und Aminen kann beispielsweise nach den in WO 97/24346 oder DE 1 795 007 oder gemäß der deutschen Anmeldung DE 10156885.1 beschriebenen Verfahren erfolgen.

Das erfindungsgemäße Verfahren wird nun in den nachfolgenden Beispielen erläutert.

### Beispiele

### a) Katalysatorherstellung (Katalysator C)

In einem beheizbaren und mit Rührwerk ausgestatteten Fälltopf werden 1,5 1 Wasser vorgelegt und auf 80°C erwärmt. In dieses Fällgefäß werden im Verlauf einer Stunde eine Metallsalzlösung bestehend aus 731 g Cu(NO₃)₂ * 2,5 H₂O und 1840 g Al(NO₃)₃* 9 H₂O in 2000 ml Wasser gleichzeitig eine 20 Gew.-% Sodalösung unter Rühren zudosiert, bis im Fällgefäß ein pH-Wert von 8 erreicht ist und weitere 15 min. bei diesem pH-Wert gerührt. Der Gesamtverbrauch an Sodalösung liegt bei 5,6 kg. Die gebildete Suspension wird abfiltriert und mit Wasser gewaschen, bis das ablaufende Waschwasser kein Nitrat (< 25 ppm) mehr enthält. Der Filterkuchen wird zunächst bei 120°C getrocknet und anschließend bei 600°C calciniert. Der so hergestellte Katalysator enthält 50 Gew.-% CuO und 50 Gew.-% Al₂O₃. 400 g dieses Katalysatorpulvers werden auf eine Korngröße von < 1 mm zerkleinert, mit 12 g Graphitpulver versetzt, intensiv durchmischt und zu Tabletten von 3 mm Durchmesser und 3 mm Höhe verpresst.

Die Katalysatoren A und B wurden in ähnlicher Weise unter Verwendung von Zinknitrat hergestellt

| | |
|---|---|
| Katalysator A: | 70 Gew.-% CuO, 25 Gew.-% ZnO, 5 Gew.-% Al₂O₃ |
| Katalysator B: | 40 Gew.-% CuO, 40 Gew.-% ZnO, 20 Gew.-% Al₂O₃ |

### b) Aktivierung der Katalysatoren A bis C

Vor dem Reaktionsbeginn wird der Katalysator in der Hydrierapparatur einer Wasserstoffbehandlung unterzogen.

Der Hydrierreaktor der Hydrierapparatur wird mit unterschiedlichen Mengen Katalysator befüllt und mit 500 NL/h N₂ bei Normaldruck durchflutet. Anschließend wird das Katalysatorbett auf die in Tabelle 1 angegebene Temperatur erhitzt und unterschiedliche Zeiten mit Gemischen von Wasserstoff und Stickstoff behandelt. Die Reihenfolge, Reduktionszeiten, Temperatur und Mischungsverhältnisse sind der nachfolgenden Tabelle 1 zu entnehmen.

**Tabelle 1**

| Kat | Zeit [min] | Temperatur [°C] | N₂/H₂-Gemische | |
|---|---|---|---|---|
| | | | N₂ [Nl/h] | H₂ [Nl/h] |
| A, C | 70 | 250 | 500 | 10 |
| | 30 | | 300 | 10 |
| | 720 | | 0 | 60 |
| B | 40 | 160 | 300 | 25 |
| | 20 | | 100 | 25 |
| | 20 | | 0 | 25 |
| | 720 | | 0 | 65 |

### c) Hydrierapparatur

Die zur Hydrierung verwendete Druckapparatur besteht aus einem Verdampfer, einem Reaktor, einem Kühler mit Quenchzulauf, einer Wasserstoffzufuhr, einer Abgasleitung und einem Kreisgasgebläse. Der Druck in der Apparatur wird konstant gehalten.

Das aufgeschmolzene MSA wird im Gleichstrom mit Wasserstoff in den vorgeheizten (245°C) Verdampfer gepumpt und verdampft. Auf den Verdampfer gelangt ebenfalls von oben eine Mischung aus frischem Wasserstoff und Kreisgas. Wasserstoff und MSA gelangen so von unten in den temperierten Reaktor. Der Reaktorinhalt besteht aus einem Gemisch aus Glasringen und Katalysator. Nach der Hydrierung verlassen die entstandenen Reaktionsprodukte zusammen mit Wasserstoff den Reaktor und werden im Kühler niedergeschlagen. Ein Teil des Kreisgases wird ausgeschleust, bevor der Rest, mit Frischwasserstoff vermischt, wieder in den Verdampfer eintritt.

Der kondensierte flüssige Reaktionsaustrag, das Abgas und das Kreisgas werden gaschromatographisch quantitativ analysiert.

### d) Hydrierung von aus n-Butan hergestelltem Maleinsäureanhydrid

### Beispiel 1

Der Reaktor der in 1c beschriebenen Hydrierapparatur wird mit 220 ml des analog zu Katalysator C hergestellten Katalysators A und 126 ml Raschigringen gefüllt. Die Aktivierung erfolgte wie in 1b beschrieben.

Als Edukt wird aus n-Butan hergestelltes Maleinsäureanhydrid eingesetzt, das 500 ppm Acrylsäure, 1500 ppm Essigsäure und 100 ppm Dibutylphthalat enthält. Die Umsetzung wird 1000 h lang durchgeführt. Dabei wird über den gesamten Zeitraum keinerlei Desaktivierung des Katalysators, d.h. kein Rückgang des Maleinsäureanhydrid-Umsatzes und/oder der GBL- und Tetrahydrofuran-Ausbeute beobachtet. In Tabelle 2 sind die Reaktionsparameter der Hydrierung und die Ergebnisse zusammengefaßt.

### Beispiel 2 a) und 2 b)

Ein analog zu Katalysator C hergestellter Katalysator B, der zu 40 % aus Kupferoxid, 40 % aus Zinkoxid und zu 20 % aus Aluminiumoxid besteht, wird in die oben beschriebene Hydrierapparatur eingebaut und wie in 1b beschrieben mit Wasserstoff vorbehandelt. Die Füllung des Reaktors bestand aus 220 ml Katalysator B und 126 ml Raschigringen. Als Edukt wurde aus n-Butan hergestelltes reines Maleinsäureanhydrid eingesetzt. Die Reaktionsparameter und Ergebnisse sind in Tabelle 2 dargestellt.

### Beispiel 3

Der Reaktor der in 1c beschriebenen Hydrierapparatur wurde mit einem Gemisch aus 80 ml Katalysator C und 80 ml Raschigringen gefüllt. Das restliche Volumen wurde mit Raschigringen angefüllt. Die Aktivierung erfolgte wie in 1b beschrieben. Als Edukt wird aus n-Butan hergestelltes Maleinsäureanhydrid eingesetzt, das 1000 ppm Acrylsäure, 1500 ppm Essigsäure und 100 ppm Dibutylphthalat enthält. Die Umsetzung wurde 1000 h lang durchgeführt. Dabei wurde über den gesamten Zeitraum keinerlei Desaktivierung des Katalysators, d.h. kein Rückgang des Maleinsäureanhydrid-Umsatzes und/oder der GBL- und Tetrahydrofuran-Ausbeute beobachtet. In Tabelle 2 sind die Reaktionsparameter der Hydrierung und die Ergebnisse zusammengefasst.

**Tabelle 2**

| Bsp. | Kat. | Temp. [°C] | Druck [bar] | Belastung [kg_{MSA}/Kg_{Kat}h] | H₂/MSA [mol/mol] | Umsatz [%] | GBL [%] | Butanol [%] | BDO¹⁾ [%] | THF [%] | BSA²) [%] | THF³) [%] |
|---|---|---|---|---|---|---|---|---|---|---|---|---|
| 1 | A | 240 | 15,3 | 0,1 | 75 | 100 | 38,5 | 3,1 | 0 | 50,5 | 0,2 | 56 |
| 2a | B | 250 | 10 | 0,24 | 90 | 100 | 66,7 | 4,8 | 3 | 20,7 | 0,13 | 23 |
| 2b | | 250 | 2 | 0,11 | 76 | 100 | 83,4 | 4,9 | 0,5 | 7 | 1,1 | 7 |
| 3 | C | 257 | 5 | 0,11 | 95 | 100 | 9,9 | 5,7 | 0 | 83,2 | 0,1 | 89 |

| | | | | | | | | | | | | |
|---|---|---|---|---|---|---|---|---|---|---|---|---|
| 1) Butandiol 2) Bernsteinsäureanhydrid 3) Bezogen auf die Summe aus GBL und THF | | | | | | | | | | | | |

### Beispiel 4

### a) Trennung von THF und GBL

Der Hydrieraustrag gemäß Beispiel 2a) mit der in Tabelle 2 angegebenen Zusammensetzung wurde zur erfindungsgemäßen Auftrennung in ein THF/Wasser-Gemisch und GBL in einer Kolonne mit 15 theoretischen Trennstufen bei 1,3 bar, absolut, einer Sumpftemperatur von 214°C und einer Kopftemperatur von 76°C destilliert. Der aus Hydrieraustrag bestehende Feedstrom wurde dabei im Massenverhältnis Sumpfprodukt : Kopfprodukt von 70 : 30 aufgetrennt.

### b) Reinigung von THF

Es wurde die durch die Figur dargestellte Destillationsapparatur verwendet. Sie besteht aus den drei Kolonnen (1), (2) und (3), von denen die Kolonne (1) 48 und die Kolonne (3) 45 theoretische Böden aufweist und bei 1,3 bar absolut betrieben wird. Kolonne (2), die bei erhöhtem Druck von 8 bar betrieben wird, hat 43 theoretische Böden.

In die Kolonne (1) wurde über die Zuleitung (4) das Kopfprodukt der THF/GBL-Kolonne, ein wasserhaltiges THF-Gemisch mit 77,2 Gew.-% THF, 18,4 Gew.-% n-BuOH, 0,3 Gew.-% MeOH, 0,5 Gew.-% EtOH, 0,5 Gew.-% PrOH, 134 ppm GBL, 110 ppm BA und 90 ppm BME zugeführt. Der Rest des Kopfproduktes sind weitere O-funktionalisierte CH-Verbindungen und größtenteils Wasser.

Gleichzeitig wurde der Kolonne (1) über Zuleitung (8) der Sumpfabzug der Kolonne (2) und über Leitung (11) der Sumpfabzug der Kolonne (3) zugeführt. Die Kolonne (1) wurde mit einem Seitenabzug (6), einem Kopfabzug (12) und einem Sumpfabzug (5) betrieben. Das Rücklaufverhältnis bezogen auf den Seitenabzug, betrug 1,1. Aus dem Sumpfabzug (5) wurde Wasser und Schwersieder abgeleitet. Der Kolonne (1) wurde als Seitenabzug (6) ein wasserhaltiges THF mit einem Wassergehalt von 4,2 Gew.-% und einer THF-Konzentration von 83 Gew.-% entnommen. Als Kopfabzug (12) wurden etwas über 0,05 Gew.-% des Zulaufs entnommen. Der Kopfabzug hatte eine THF-Konzentration von 66 % und einen Methanolgehalt von 30 Gew.-%. Der Seitenabzug (6) der ersten Kolonne wurde unter Druckerhöhung der zweiten Kolonne (2) zugeführt, die bei 8 bar betrieben wurde. Der Kopfabzug (7) der zweiten Kolonne, der praktisch alles Wasser und den Großteil der engsiedenden Komponenten enthielt, wurde zur ersten Kolonne (1) zurückgeführt. Ein stark THF angereicherter Strom wird flüssig als Seitenabzug (9) kurz über dem Sumpf entnommen und in Kolonne (3) weitergeleitet. Der Sumpfabzug von Kolonne (2), der sich als praktisch wasserfrei erwies, wurde über Leitung (8) rückgeführt. Über Kopf (10) der dritten Kolonne (3) wurde Reinst-THF abzogen, während das Sumpfprodukt über eine Pumpe in Kolonne (1) rückgeführt wird.

Die Reinheit des so hergestellten THF betrug 99,97 Gew.-%. Die Menge an den Nebenprodukten n-Butyraldehyd und n-Butylmethylether betrug jeweils nur 0,001 Gew.-%.

### Beispiel 5

In Beispiel 4.1 gewonnenes Roh-GBL wurde gemäß WO 97/24.346, Beispiel 5, ohne weitere Reinigung bei 290°C mit Methylamin umgesetzt. Die gaschromatographisch ermittelte N-Methylpyrrolidon-Ausbeute betrug 98,5 % (bezogen auf eingesetztes GBL).

## Patentansprüche

1. Verfahren zur gleichzeitigen Herstellung von gegebenenfalls alkylsubstutierten THF und Pyrrolidonen durch katalytische Hydrierung von C₄-Dicarbonsäuren und/oder deren Derivaten in der Gasphase in Gegenwart von Kupfer-enthaltenden Katalysatoren und Umsetzung von GBL mit Ammoniak oder primären Aminen zu Pyrrolidonen, **dadurch gekennzeichnet, dass** man
a) C₄-Dicarbonsäuren und/oder deren Derivate in der Gasphase bei 200 bis 300°C, 0,1 bis 100 bar, Katalysator-Belastungen von 0,01 bis 1 kg Edukt/l Katalysator * Stunde und Edukt/Wasserstoff-Molverhältnissen von 20 bis 800 in Gegenwart von<70 Gew.-% Kupferoxid und > 30 Gew.-% Trägermaterial ausgewählt aus Aluminiumoxid oder Aluminiumoxid und Zinkoxid enthaltenden Trägerkatalysatoren zu Gemischen aus THF und GBL hydriert,
b) den erhaltenen Hydrieraustrag durch Destillation in ein THF/Wasser-Gemisch als Kopfprodukt und ein GBL enthaltendes Sumpfprodukt auftrennt,
c) das THF/Wasser-Gemisch aus Stufe b) in einer aus drei Kolonnen bestehenden Destillationsanordnung trennt, in dem man Wasser aus dem Sumpf der ersten Kolonne abzieht, wasserhaltiges THF von der zweiten in die erste Kolonne zurückführt, einen Seitenstrom der ersten in die zweite Kolonne leitet, das Sumpfprodukt der dritten Kolonne in die erste Kolonne zurückführt, am Kopf der ersten Kolonne ein Destillat entnimmt, wobei ein Seitenabzug der zweiten Kolonne in die dritte Kolonne geleitet wird und das reine THF als Kopfprodukt der dritten Kolonne erhält,
d) aus dem GBL enthaltenden Sumpfprodukt aus Stufe b) mit Ammoniak oder Aminen zu entsprechenden Pyrrolidonen umsetzt.

2. Verfahren nach Anspruch 1, **dadurch gekennzeichnet, dass** aus dem GBL enthaltenden Sumpfprodukt aus Stufe b) vor der Umsetzung mit Ammoniak oder Aminen durch Destillation GBL gewonnen wird.

3. Verfahren nach Anspruch 1 oder 2, **dadurch gekennzeichnet, dass** Maleinsäureanhydrid als Edukt in die Reaktion eingesetzt wird.

4. Verfahren nach Anspruch 1 bis 3, **dadurch gekennzeichnet, dass** das Verfahren an einem Katalysator enthaltend < 70 Gew.-% CuO und > 30 Gew.- eines oxidischen Trägers mit sauren Zentren und Katalysatorbelastungen von 0,01 bis 1,0 Edukt/l Katalysator * Stunde durchgeführt wird.

5. Verfahren nach den Ansprüchen 1 bis 4, **dadurch gekennzeichnet, dass** der oxidische Träger Al₂O₃ oder Al₂O₃/ZnO im Gew.-Verhältnis 20:1 bis 1 : 20 ist.

## Claims

1. A process for coproducing alkyl-substituted or unsubstituted THF and pyrrolidones by catalytically hydrogenating C₄-dicarboxylic acids and/or derivatives thereof in the gas phase in the presence of copper-comprising catalysts and reacting GBL with ammonia or primary amines to give pyrrolidones, which comprises
a) hydrogenating C₄-dicarboxylic acids and/or derivatives thereof in the gas phase at from 200 to 300°C, from 0.1 to 100 bar, catalyst hourly space velocities of from 0.01 to 1 kg of reactant/l of catalyst * hour and reactant/hydrogen molar ratios of from 20 to 800 in the presence of supported catalysts comprising < 70% by weight of copper oxide and > 30% by weight of support material selected from aluminum oxide or aluminum oxide and zinc oxide to give mixtures of THF and GBL,
b) separating the hydrogenation effluent obtained by distillation into a THF/water mixture as the top product and a GBL-comprising bottom product,
c) separating the THF/water mixture from step b) in a distillation facility consisting of three columns by withdrawing water from the bottom of the first column, recycling water-containing THF from the second into the first column, passing a side stream of the first into the second column, recycling the bottom product of the third column into the first column and withdrawing a distillate at the top of the first column, wherein a side stream of the second column is passed into the third column and the pure THF is obtained as the top product of the third column,
d) reacting from the GBL-comprising bottom product from step b) with ammonia or amines to give corresponding pyrrolidones.

2. The process according to claim 1, wherein GBL is recovered from the GBL-comprising bottom product from step b) by distillation before the reaction with ammonia or amines.

3. The process according to claim 1 or 2, wherein the reactant used in the reaction is maleic anhydride.

4. The process according to claims 1 to 3, wherein the process is operated over a catalyst comprising < 70% by weight of CuO and > 30% by weight of an oxidic support having acidic sites and at catalyst hourly space velocities of from 0.01 to 1.0 kg of reactant/l of catalyst * hour.

5. The process according to any of claims 1 to 4, wherein the oxidic support is Al₂O₃ or Al₂O₃/ZnO in a weight ratio of from 20:1 to 1:20.

## Revendications

1. Procédé de préparation simultanée de THF éventuellement substitué par de l'alkyle et de pyrrolidones par hydrogénation catalytique d'acides dicarboxyliques en C₄ et/ou de leurs dérivés dans la phase gazeuse, en présence de catalyseurs contenant du cuivre, et réaction de GBL avec de l'ammoniac ou des amines primaires pour former des pyrrolidones, **caractérisé en ce que**
a) on hydrogène des acides dicarboxyliques en C₄ et/ou leurs dérivés dans la phase gazeuse à 200 jusqu'à 300°C, à une pression de 0,1 à 100 bars, avec des alimentations en catalyseur de 0,01 à 1 kg de matière de départ/litre de catalyseur * heure et dans des proportions molaires de matière de départ/hydrogène de 20 à 800, en présence de catalyseurs sur support contenant < 70 % en poids d'oxyde de cuivre et > 30 % en poids de matière de support choisie parmi de l'oxyde d'aluminium ou de l'oxyde d'aluminium et de l'oxyde de zinc, pour former des mélanges de THF et de GBL,
b) par distillation, on sépare le produit de sortie de l'hydrogénation obtenu en un mélange de THF/eau comme produit de tête et un produit de fond contenant du GBL,
c) on sépare le mélange de THF/eau de l'étape b) dans un appareillage de distillation qui est constitué de trois colonnes et dans lequel on soutire l'eau du fond de la première colonne, on recycle du THF contenant de l'eau de la deuxième colonne dans la première, on conduit un courant latéral de la première colonne dans la deuxième, on recycle le produit de fond de la troisième colonne dans la première colonne, on prélève à la tête de la première colonne un distillat, un soutirage latéral de la deuxième colonne étant conduit dans la troisième colonne et le THF pur étant obtenu sous la forme de produit de tête de la troisième colonne,
d) on fait réagir le produit de fond contenant du GBL de l'étape b) avec de l'ammoniac ou des amines pour former des pyrrolidones correspondantes.

2. Procédé suivant la revendication 1, **caractérisé en ce que**, à partir du produit de fond contenant du GBL de l'étape b), on produit du GBL par distillation avant la réaction avec de l'ammoniac ou des amines.

3. Procédé suivant la revendication 1 ou 2, **caractérisé en ce que** de l'anhydride d'acide maléique est introduit dans la réaction comme matière de départ.

4. Procédé suivant les revendications 1 à 3, **caractérisé en ce que** le procédé est effectué sur un catalyseur contenant < 70 % en poids de CuO et > 30 % en poids d'un support oxydé ayant des centres acides et des alimentations en catalyseur de 0,01 à 1,0 kg de matière de départ/litre de catalyseur * heure.

5. Procédé suivant les revendications 1 à 4, **caractérisé en ce que** le support oxydé est du Al₂O₃ ou du Al₂O₃/ZnO dans un rapport pondéral de 20/1 à 1/20.
